(19) Europäisches Patentamt
European Patent Office
Office européen des brevets

(11) Publication number : **0 445 700 B1**

(12) # EUROPEAN PATENT SPECIFICATION

(45) Date of publication of patent specification : **13.07.94 Bulletin 94/28**

(51) Int. Cl.$^5$ : **A61K 7/48,** A61K 7/06

(21) Application number : **91103231.6**

(22) Date of filing : **04.03.91**

(54) **Emollient durability enhancing siloxanes.**

(30) Priority : **05.03.90 US 488549**
**05.03.90 US 488163**
**05.03.90 US 488159**

(43) Date of publication of application : **11.09.91 Bulletin 91/37**

(45) Publication of the grant of the patent : **13.07.94 Bulletin 94/28**

(84) Designated Contracting States : **DE FR GB**

(56) References cited :
**EP-A- 0 078 597**
**EP-A- 0 095 238**

(56) References cited :
**EP-A- 0 174 097**
**WO-A-89/04161**
**US-A- 4 501 619**
**US-A- 4 559 227**

(73) Proprietor : **DOW CORNING CORPORATION**
**P.O. Box 1767**
**Midland Michigan 48686-0994 (US)**

(72) Inventor : **Klimisch, Helen Marie**
**472 E. Olson Road**
**Midland, Michigan (US)**

(74) Representative : **Spott, Gottfried, Dr. et al**
**Spott Weinmiller & Partner**
**Sendlinger-Tor-Platz 11**
**D-80336 München (DE)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the enhancement of the durability of emollient materials with certain organosilicon compounds. More particularly, the durability of mineral oil, for example, on human skin is enhanced by mixing the emollient with one of aminofunctional, amidofunctional, or carboxyfunctional, polysiloxanes.

From US-A 4 501 619 it was known to use an aqueous emulsion for the treatment of fibres which comprises a primary surfactant and a carboxy-functional polyorganosiloxane.

EP-A 0 095 238 discloses a composition used to condition hair which comprises an aminofunctional polysiloxane together with a surfactant.

Also from EP-A 0 174 097 a method is known wherein an aminoalkyl-substituted polyorganosiloxane is used together with an organic carboxylic acid for hair setting.

WO-A-89 04 161 discloses a polysiloxane cosmetic composition which contains a mixture of two types of polysiloxanes which are applied to the hair or to the skin. The composition can be removed with a surface-active agent.

From EP-A 0 078 597 compositions are known which are used to groom hair comprising an organopolysiloxane which has at least one alkylamino substituent and at least one substituent selected from the group consisting of oxyalkylene, polyoxyalkylene and hydroxyalkyl groups.

This invention relates to a method of enhancing the durability of an emollient material on a substrate by forming a mixture of an emollient material and an effective amount of an organosilicon compound, and applying the mixture to the substrate to be treated. The organosilicon compound is either an aminofunctional, amidofunctional, or carboxyfunctional, polysiloxane.

Preferred embodiments of the present invention include an aminofunctional polysiloxane having the formula $Me_3SiO(Me_2SiO)_x(MeRSiO)_ySiMe_3$ in which Me is methyl; R is the functional group $-(C_4H_8)NH(CH_2)_2NH_2$; x is an integer from fifty to one thousand; and y is an integer from one to fifty preferably one to twenty. The amidofunctional polysiloxane preferably has the formula $Me_3SiO(Me_2SiO)_x(MeRSiO)_ySiMe_3$ in which Me is methyl; R is the functional group $-(C_4H_8)NH(CH_2)_2NHCOCH_3$; x is an integer from fifty to one thousand preferably fifty to one hundred; and y is an integer from one to fifty preferably one to ten. Preferably, the carboxyfunctional polysiloxane compound has the formula $Me_3SiO(Me_2SiO)_x(MeRSiO)_ySiMe_3$ in which Me is methyl; R is the functional group $-(C_3H_6)COOH$; x is an integer from fifty to one thousand preferably one hundred to three hundred; and y is an integer from one to fifty.

The emollient and the polysiloxane may be present in the mixture in the ratio of four to one, more preferably two to one. The mixture of the emollient and the polysiloxane may, if desired, include a volatile cyclic siloxane solvent, and the solvent can be a low viscosity polydimethylcyclosiloxane fluid, for example, which is a mixture of cyclic tetramers and pentamers and having a viscosity of about 2.5 mm $^{e}/_{s}$ (centistokes) measured at 25°C. The mixture should contain about five to ten percent by weight of the emollient and the polysiloxane in the volatile cyclic siloxane solvent. The emollient may be a material such as mineral oil, mink oil, lanolin oil, and petrolatum.

In accordance with the present invention, there is provided a concept which relates to the enhancement of the durability of emollient materials with certain organosilicon compounds. The durability on human skin is enhanced by mixing an emollient with either one of an aminofunctional, amidofunctional, or carboxyfunctional, polysiloxanes. These aminofunctional, amidofunctional, and carboxyfunctional, organosilicon compounds are well known in the prior art, and such compounds as well as methods for preparing these compounds can be found in U.S. Patent No. 4,477,514, which shows carboxyfunctional siloxanes; U.S. Patent No. 4,559,227, which shows aminofunctional siloxanes; and U.S. Patent No. 4,848,981, which shows the amidofunctional siloxanes, of the present invention. A brief description of these functional siloxanes is set forth hereinbelow.

The amine functional siloxane polymer has the formula

$$R'_{3-z}Q_zSiO[R'_2SiO]_x[R'QSiO]_ySiQ_zR'_{3-z}$$

wherein R′ denotes an alkyl group of 1 to 4 carbons or a phenyl group, with the proviso that at least 50 percent of the total R′ groups are methyl; Q denotes an amine functional substituent of the formula —R″Z, wherein R″ is a divalent alkylene radical of 3 to 6 carbon atoms or a radical of the formulation $-CH_2CH_2CH_2OCH_2-CHOHCH_2-$ and Z is a monovalent radical selected from the group consisting of $—NR_2'''$, $—NR'''(CH_2)_nNR_2'''$; and

$$NR'''(CH_2)_nN(R''')\overset{\overset{O}{\|}}{C}R''''$$

wherein R‴ denotes hydrogen or an alkyl group of 1 to 4 carbons, R⁗ denotes an alkyl group of 1 to 4 carbons and n is a positive integer from 2 to 6; z has a value of 0 or 1; x has an average value of 25 to 3000; y has an average value of 0 to 100 when z is 1, and y has an average value of 1 to 100 when z is 0.

The amidofunctional polysiloxane is a triorganosiloxane-endblocked polydiorganosiloxane having an average of 50 to 1000 siloxane units per molecule with an average of 1 to 50 of the siloxane unites per molecule being amide-containing siloxane units. The amide-containing siloxane units bear a substituent of the formula

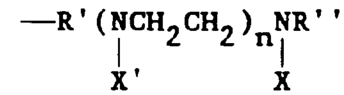

wherein n is 0 or 1, R′ denotes an alkylene radical of 3 to 6 carbon atoms, and R″ denotes a hydrogen radical or an alkyl radical of 1 to 6 carbon atoms, X denotes an acyl radical of the formula

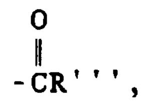

X′ denotes a hydrogen radical or X, and R‴ denotes an alkyl radical of 1 to 4 carbon atoms and substantially all other organic substituents in the polydiorganosiloxane being methyl groups.

The amidofunctional silicone component in accordance with this invention consists essentially of a triorganosiloxane-endblocked polydiorganosiloxane which contains amidoalkyl substituents. Triorganosiloxane-endblocked polydiorganosiloxanes (amidofunctional silicone) consist essentially of terminal triorganosiloxane units of the formula $R_3SiO_{1/2}$ and backbone diorganosiloxane units of the formula $R_2SiO_{2/2}$. Trace amounts of other siloxane units in amidofunctional silicone, such as $SiO_{4/2}$ and $RSiO_{3/2}$, which are normally present as impurities in commercial polydiorganosiloxanes may be present. Preferably there are no $SiO_{4/2}$ units or $RSiO_{3/2}$ units in the amidofunctional silicones.

The R radicals of the above siloxane units are substantially either amide-containing radicals of the formula

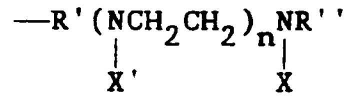

or methyl radicals. Minor amounts of other organic substituents which are normally present as impurities in commercial polydiorganosiloxanes may be present. It should be understood, for example, that the amidofunctional silicones of this invention are often prepared by acylation of corresponding aminofunctional silicones. Consequently, the amidofunctional silicones may also contain residual aminofunctional siloxane units. For example, siloxane unites such as $H_2NCH_2CH_2NHCH_2CH(CH_3)CH_2SiO_{2/2}$ or $H_2NCH_2CH_2CH_2SiO_{2/2}$ may also be present in the amidofunctional silicones useful in this invention. However, for the purposes of this invention it is preferred to employ silicone oils that do not contain significant levels (more than 25 percent of the number of amidofunctional substituents) of the unmodified aminofunctional siloxane units.

In the formula for the amide-containing radicals, R′ denotes an alkylene radical of 3 to 6 carbon atoms, such as $-CH_2CH_2CH_2-$, $-CH_2CH_2CH_2CH_2-$, $-CH_2CH(CH_3)CH_2-$, $-CH_2CH_2CH_2CH_2CH_2-$, and $-CH_2CH(CH_2CH_3)CH_2-$. Amidofunctional silicones wherein the silicon bonded, amide-containing radicals have a trimethylene radical or an alkylated trimethylene radical, such as $-CH_2CH(CH_3)CH_2-$, as the R′ radical are preferred because of ease of synthesis and availability.

R″ denotes a hydrogen radical, which is a preferred R″ radical, or an alkyl radical of 1 to 6 carbon atoms, such as methyl, ethyl, propyl, butyl, and isobutyl.

In the formula for the amide-containing radicals, n has a value of 0 or 1, so that the radical may contain one or two nitrogen atoms. X denotes an acyl radical of the formula

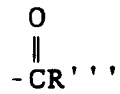

and X′ denotes a hydrogen radical or X. In the acyl radical, R‴ denotes an alkyl radical of 1 to 4 carbon atoms such as methyl, ethyl, propyl, isopropyl, or butyl.

In accordance with the above, triorganosiloxane-endblocked polydiorganosiloxanes preferred for use in the method of this invention consists essentially of siloxane units selected from the following:

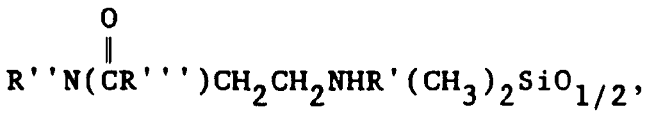

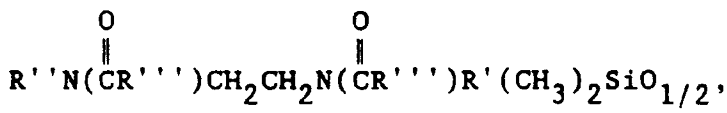

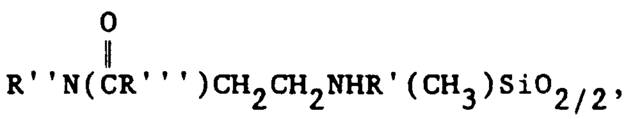

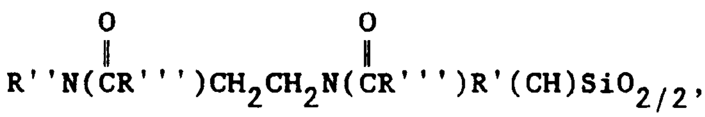

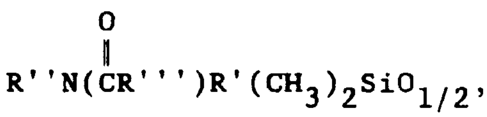

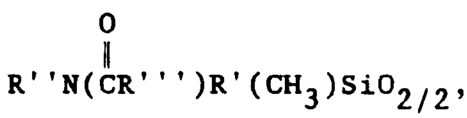

$(CH_3)_3SiO_{1/2}$, and $(CH_3)_2SiO_{2/2}$ where R′, R″, and R‴ have the same meanings as described above. It should be understood that any of the siloxane units having non-acylated nitrogen atoms can also be present in their salt form. It is well known that the salt form occurs when such polymers are neutralized by acids such as mineral acids or carboxylic acids.

The silicone polymers of this invention may contain amide-containing siloxane units of the formula

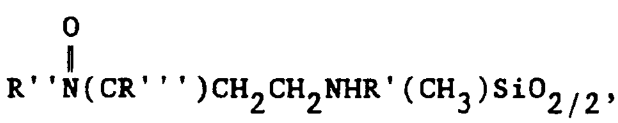

wherein R′, R″, and R‴ have the same meanings as described above. These amide-containing units have a ratio of acyl groups to nitrogen atoms of about 0.5.

The carboxyfunctional silicones of the invention have the formula

$$QMe_2SiO(Me_2SiO)_x(MeRSiO)_ySiMe_2Q$$

wherein Me is a methyl radical, R is a carboxyfunctional radical, said carboxyfunctional radical being selected

from the group consisting of carboxyalkyl radicals and carboxythioalkyl radicals, Q is selected from the group consisting of R, Me and OH groups, x has a value of 1 to 1000, and y has a value of 1 to 100.

As referred to herein, a carboxyfunctional radical is a monovalent radical which contains the —COOH radical, and is attached to a silicon atom of the main molecular chain by a divalent linking group. Direct attachment to the silicon atom is through a silicon to carbon bond.

Divalent linking groups contemplated for use in the present invention are either alkylene groups containing from 2 to 9 carbon atoms, or thioalkylene groups, containing 2 to 8 carbon atoms and one sulfur atom present as a thioether group.

Those carboxyfunctional radicals wherein the divalent linking group is an alkylene group are referred to herein as carboxyalkyl radicals; those carboxyfunctional radicals wherein the divalent linking group is a thioalkylene group are referred to herein as carboxythioalkyl radicals.

Specific examples of carboxyalkyl radicals include, but are not limited to $—CH_2CH_2COOH$, $—CH_2CH(CH_3)COOH$, $—CH_2CH(C_2H_5)CH_2COOH$, $—CH_2CH(CH_3)CH(CH_3)CH_2COOH$, and the like. The $—CH_2CH(CH_3)COOH$ radical is a preferred carboxyalkyl radical for the practice of the present invention.

Specific examples of carboxythioalkyl radicals include, but are not limited to $-CH_2CH_2SCOOH$, $-(CH_2)_3SCOOH$, $-CH_2CH(CH_3)SCH_2COOH$, $—CH_2CH_2SCH_2COOH$, $—CH_2CH(C_2H_5)SCH_2COOH$, and the like. The $—CH_2CH_2SCH_2COOH$ radical is a preferred carboxythioalkyl radical for the practice of the present invention.

Examples of emollients and moisturizers which may be used in this invention include straight, branched or cyclic hydroxy compounds such as alcohols containing 1 to 30 carbon atoms; straight, branched, or cyclic carboxylic acids containing 1 to 31 carbon atoms; acid esters containing $C_1$ to $C_{30}$ carboxylic acids esterfied with $C_1$ to $C_{30}$ alcohols; alcohol ethers containing 1 to 30 carbon atoms; alkanes of the formula $H—(CH_2)n-H$, wherein n is 5 to 30; and siloxanes. Examples of such functional materials include 2-ethylhexyl oxystearate; arachidyl propionate; 2-ethylhexyl adipate; isopropyl myristate; ethanol; stearyl alcohol; propylene glycol; propionic acid; stearic acid; polyoxypropylene cetyl alcohol; polyoxypropylene lanolin alcohol; Carbowax® 300; petroleum jelly; mineral oil; aliphatic hydrocarbons such as mineral spirits; lanolin and lanolin derivatives such as acetylated lanolin and isopropyl lanolate; hexamethyldisiloxane; cyclic polydimethylsiloxane; linear polydimethylsiloxane; polypheylmethylsiloxane; and poly dimethyl/trimethylsiloxane. Other phenyl, ethyl and vinyl substituted polysilanes may also be included in the products of this invention.

In order to illustrate the durability enhancement of the siloxanes of the present invention, durability enhancement data was collected for a variety of emollients. A soap washing test procedure was used in order to measure the influence of the silicones on the durability of the various emollients. The emollients considered were mineral oil, mink oil, lanolin oil, and petrolatum. The test procedure was used to measure silicone substantivity on human skin. Specifically, the method was based on Attenuated Total Reflectance/Fourier Transform Infrared Spectrophotometric (ATR/FTIR) analysis, in which prism skin studies were conducted and analyzed based on the reflection of energy at the interface. Instrumentation included a NICOLET Model 20DX FTIR system, and a HARRICK Scientific Skin Analyzer. The ATR studies involved contact of the skin sample and prism. A hydration procedure was employed in order to increase the softness and flexibility of the skin surface which resulted in a less variable contact between the skin and prism. This hydration procedure included placing a water soaked towel against the skin test site for one minute prior to actual spectra collection. A skin test site selected was an area of about eighty square centimeters, and about ten to twelve milligrams of each solution tested was applied to the skin test site area in the form of a thin film using a small paint brush. From the data collected, it was possible to calculate percentages of ingredients remaining on the skin following various soap wash sequences. The soap employed was a 0.5 weight percent solution of IVORY bar soap, and a soap rub is defined as two passes over the test area with the soap solution cupped in the palm of the hand. One soap wash procedure included fifteen soap rubs and ten rinse rubs under cool running tap water. The test site was the volar forearm. The test solutions were applied to the skin test site on the forearm in the form of a mixture of the various silicones and emollients, dissolved in a volatile silicone fluid of low viscosity, such as polydimethylcyclosiloxane which is a mixture of tetramer and pentamer having a viscosity of about 2.5 centistokes measured at 25°C. The solution contained five to ten percent by weight of the mixture in the solvent. The solvent was allowed to evaporate from the volar forearm region for fifteen to thirty minutes prior to the institution of the measurement procedures. The site was hydrated as noted above and initial spectrum was collected. The data included tests conducted with and without the presence of the various silicones in the test mixture, and at least two test runs were conducted for each mixture.

A simplified test procedure is illustrated as follows. A test area on the forearm was marked, and the test area was washed with the soap solution using fifteen rubs, followed by rinsing with ten rubs under cool running water. Excess moisture was blotted from the forearm with a towel. After one minute, the skin was hydrated for one minute using a towel saturated with water which was held loosely over the test area. Excess moisture was blotted, and at the end of thirty seconds a background scan was run. The test mixture was applied to the

skin test area and the solvent allowed to evaporate. The skin was again hydrated for one minute and excess moisture was blotted off. After thirty seconds, a scan was run of the test area which represented an Initial Condition. The test area was washed with the soap solution using fifteen rubs followed by ten rinses, and the excess moisture was blotted off. After one minute, the skin was hydrated for one minute, blotted, and at the end of thirty seconds, a scan was run of the test area which represented a First Soap Wash Condition. Similar steps were repeated for second, third, and fourth, soap wash conditions. Baselines for infrared bands were defined and band heights were measured. The percent ingredient remaining on the skin was calculated using this data.

The following tables set forth the results of the foregoing procedures, and illustrate the concept of the present invention of enhancing the durability of various emollients with certain functional silicones. The tables indicate that the functional silicones enhance emollient durability and therefore provide a viable solution to dry, chapped, and rough skin, which results when the emollients are removed by washing. The siloxanes are soap wash resistant and have shown minimal or no dermal irritation. The carboxyfunctional siloxane is known to possess the least dermal irritation of the siloxane types tested. The functional silicones used in the tables conform to the formula $Me_3SiO(Me_2SiO)_x(MeRSiO)_ySiMe_3$ in which Me is methyl and R is the functional group. Specifics of the R group and values of the integers x and y are set forth in the tables where appropriate. Unless otherwise indicated, the mixtures of emollient and silicone were in a ratio of four to one, and the mixtures were delivered in form of mixtures including a volatile cyclic siloxane. The compositions of the present invention may contain other adjuvants such as perfumes, fragrances, and preservatives, provided the addition of the adjuvant to the composition would not materially affect the basic and novel characteristics of the composition and would not materially change its fundamental characteristics.

TABLE I

EMOLLIENTS - NO SILICONE
PERCENT REMAINING

| Test Condition | Mink Oil | Lanolin Oil | Mineral Oil | Petrolatum |
|---|---|---|---|---|
| Initial | 100 | 100 | 100 | 100 |
| 1st wash | 32 | 33 | 32 | 44 |
| 2nd wash | 19 | 21 | 16 | 34 |
| 3rd wash | 14 | 15 | 6 | 19 |
| 4th wash | --- | --- | 4 | 16 |
| 5th wash | --- | --- | 2 | 13 |

TABLE II

COMPOUNDS USED
AMINE AND AMIDE FUNCTIONAL SILICONES

| Silicone | R-Group | M%R | x | y | x/y |
|---|---|---|---|---|---|
| A | $iBuNH(CH_2)_2NH_2$ | 1 | 97 | 1 | 91/1 |
| B | $iBuNH(CH_2)_2NH_2$ | 2 | 96 | 2 | 48/1 |
| C | $iBuNH(CH_2)_2NH_2$ | 5 | 188 | 10 | 19/1 |
| D | $iBuNH(CH_2)_2NH_2$ | 0.7 | 296 | 2 | 148/1 |
| E | $iBuNH(CH_2)_2NH_2$ | 0.5 | 445.8 | 2.2 | 203/1 |
| F | $iBuNH(CH_2)_2NH_2$ | 1.7 | 440.4 | 7.6 | 58/1 |
| G | $iBuNH(CH_2)_2NH_2$ | 0.25 | 796 | 2 | 398/1 |
| H | $iBuNH(CH_2)_2NHCOCH_3$ | 2 | 96 | 2 | 48/1 |

TABLE III

PERCENT REMAINING
MINERAL OIL AND SILICONES

| | SILICONES | | | | | | | |
|---|---|---|---|---|---|---|---|---|
| Test Condition | H | A | B | C | D | E | F | G |
| INITIAL | 100 | 100 | 100 | 100 | 100 | 100 | 100 | 100 |
| 1st wash | 46 | 56 | 53 | 47 | 46 | 46 | 50 | 52 |
| 2nd wash | 28 | 38 | 35 | 35 | 40 | 32 | 34 | 41 |
| 3rd wash | 22 | 32 | 28 | 30 | 32 | 32 | 28 | 32 |
| 4th wash | 20 | 30 | 24 | 27 | 26 | 26 | 21 | 30 |
| 5th wash | 17 | 25 | 20 | 24 | 22 | 25 | 20 | 19 |

TABLE IV

PERCENT REMAINING
MINERAL OIL AND SILICONES

| Test Condition | Polydimethylsiloxane (1) 100DP | 550DP | 6800DP | H | Average A-G |
|---|---|---|---|---|---|
| INITIAL | 100 | 100 | 100 | 100 | 100 |
| 1st wash | 35 | 35 | 45 | 46 | 50 |
| 2nd wash | 24 | 20 | 37 | 28 | 36 |
| 3rd wash | 20 | 18 | 31 | 22 | 31 |
| 4th wash | 18 | 16 | 27 | 20 | 26 |
| 5th wash | 14 | --- | 24 | 17 | 22 |

(1): 100 DP is 350 $mm^2/s$ (Centistoke) fluid.

550 DP is hydroxy endblocked fluid with partial trimethyl capping.

6800 DP is a siloxane gum.

TABLE V

% Mineral Oil Remaining

| Sample Number | Polymer Description | Initial Condition | 1st Wash | 2nd Wash | 3rd Wash | 4th Wash | 5th Wash |
|---|---|---|---|---|---|---|---|
| 1 | No Silicone | 100 | 32 | 16 | 6 | 4 | 2 |
| 2 | H | 100 | 56 | 34 | 25 | 20 | 14 |
| 3 | A | 100 | 40 | 26 | 18 | 16 | 14 |
| 4 | B | 100 | 52 | 27 | 18 | 19 | 13 |
| 5 | C | 100 | 40 | 28 | 25 | 18 | 14 |
| 6 | D | 100 | 58 | 36 | 30 | 17 | 8 |
| 7 | E | 100 | 66 | 41 | 34 | 26 | 24 |
| 8 | F | 100 | 58 | 37 | 28 | 17 | 15 |
| 9 | G | 100 | 33 | 30 | 18 | 20 | 5 |

TABLE VI

Compounds Used
Carboxyfunctional Silicones

| Reference | R-Group | M%R | x | y | x/y |
|---|---|---|---|---|---|
| J | iPr COOH | 3 | 201.7 | 6.3 | 32/1 |
| K | iPr COOH | 0.7 | 296 | 2 | 148/1 |
| L | iPr COOH | 3.3 | 288 | 10 | 29/1 |
| M | iPr COOH | 15 | 253 | 45 | 6/1 |

TABLE VII

Percent Remaining
Mineral Oil and Silicone

| Test Condition | J | K | L |
|---|---|---|---|
| INITIAL | 100 | 100 | 100 |
| 1st Wash | 44 | 42 | 29 |
| 2nd Wash | 30 | 33 | 19 |
| 3rd Wash | 24 | 28 | 14 |
| 4th Wash | 21 | 26 | 13 |
| 5th Wash | 19 | 22 | 15 |

TABLE VIII

Mineral Oil and Silicone
Percent Silicone Remaining

| Test Condition | Polydimethylsiloxane 100DP | 550DP | 6800DP | H | A-G Amino Avg. | J-K Carboxylic Acid Avg. |
|---|---|---|---|---|---|---|
| INITIAL | 100 | 100 | 100 | 100 | 100 | 100 |
| 1st Wash | 35 | 35 | 45 | 46 | 50 | 43 |
| 2nd Wash | 24 | 20 | 37 | 28 | 36 | 32 |
| 3rd Wash | 20 | 18 | 31 | 22 | 31 | 26 |
| 4th Wash | 18 | 16 | 27 | 20 | 26 | 24 |
| 5th Wash | 14 | -- | 24 | 17 | 22 | 20 |

TABLE IX

Mineral Oil and Silicone
% Mineral Oil Remaining

| Test Condition | No Sil. | J | K | L |
|---|---|---|---|---|
| INITIAL | 100 | 100 | 100 | 100 |
| 1st Wash | 32 | 47 | 52 | 32 |
| 2nd Wash | 16 | 30 | 36 | 20 |
| 3rd Wash | 6 | 19 | 24 | 13 |
| 4th Wash | 4 | 13 | 22 | 6 |
| 5th Wash | 2 | 8 | 12 | 4 |

TABLE X

Two to One
Mink Oil and Silicone

% Mink Oil Remaining

| Test Condition | No Sil. | J | Carboxylic Acid K | L |
|---|---|---|---|---|
| INITIAL | 100 | 100 | 100 | 100 |
| 1st Wash | 32 | 46 | 45 | 30 |
| 2nd Wash | 19 | 28 | 28 | 17 |
| 3rd Wash | 14 | 26 | 25 | 10 |

The mixture of emollient and silicone can be delivered to the skin in the form of emulsions, microemulsions, solutions, dispersions, lotions, gels, aerosols, solid sticks, ointments, and creams.

## Claims

1. Use of a mixture of an emollient material selected from mineral oil, mink oil, lanolin oil and petrolatum, and an effective amount of an organosilicon compound, and applying the mixture to the substrate to be treated, the organosilicon compound being selected from the groups consisting of (a) aminofunctional polysiloxanes having the formula

$$R_{3-z}'Q_zSiO[R_2'SiO]_x[R'QSiO]_ySiQ_zR_{3-z}'$$

wherein R′ denotes an alkyl group of 1 to 4 carbons or a phenyl group, with the proviso that at least 50 percent of the total R′ groups are methyl; Q denotes an amine functional substitutent of the formula - R″Z, wherein R″ is a divalent alkylene radical of 3 to 6 carbon atoms or a radical of the formula $-CH_2CH_2CH_2OCH_2-CHOHCH_2-$ and Z is a monovalent radical selected from the group consisting of $-NR_2'''$, $-NR'''(CH_2)_nNR_2'''$; and

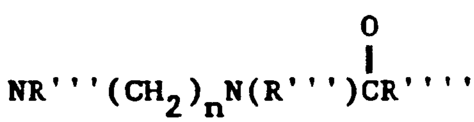

wherein R‴ denotes hydrogen or an alkyl group of 1 to 4 carbons, R⁗ denotes an alkyl group of 1 to 4 carbons and n is a positive integer from 2 to 6; z has a value of 0 or 1; x has an average value of 25 to 3000; y has an average value of 0 to 100 when z is 1, and y has an average value of 1 to 100 when z is 0, (b) amidofunctional polysiloxanes having an average of 50 to 1000 siloxane units per molecule with an average of 1 to 50 of the siloxane units per molecule being amide-containing siloxane units bearing a substituent of the formula

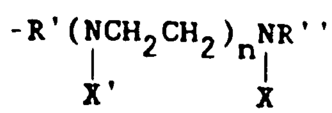

wherein n is 0 or 1, R′ denotes an alkylene radical of 3 to 6 carbon atoms, and R″ denotes a hydrogen radical or an alkyl radical of 1 to 6 carbon atoms, X denotes an acyl radical of the formula

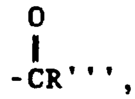

X′ denotes a hydrogen radical or X, and R‴ denotes an alkyl radical of 1 to 4 carbon atoms and substantially all other organic substituents in the polysiloxane being methyl groups, and (c) carboxyfunctional polysiloxanes having the formula

$$QMe_2SiO(Me_2SiO)_x(MeRSiO)_ySiMe_2Q$$

wherein Me is a methyl radical, R is a carboxyfunctional radical, said carboxyfunctional radical being selected from the group consisting of carboxyalkyl radicals and carboxythioalkyl radicals, Q is selected from the group consisting of R, Me and OH groups, x has a value of 1 to 1000, and y has a value of 1 to 100, for enhancing the durability of the emollient on the human skin.

2. The use of claim 1 in which the mixture of the emollient and the polysiloxane includes a volatile cyclic siloxane solvent.

## Patentansprüche

1. Verwendung einer Mischung eines weichmachenden Materials ausgewählt aus Mineralöl, Nerzöl, Lanolinöl und Vaseline und einer wirksamen Menge einer Organosiliziumverbindung und Auftragen der Mischung auf das zu behandelnde Substrat, wobei die Organosiliziumverbindung ausgewählt ist aus den Gruppen bestehend aus

   (a) aminofunktionellen Polysiloxanen mit der Formel

   $$R_{3-z}'Q_zSiO[R_2'SiO]_x[R'QSiO]_ySiQ_zR_{3-z}'$$

   worin R′ eine Alkylgruppe mit 1 bis 4 Kohlenstoffatomen oder eine Phenylgruppe bedeutet, mit dem Vorbehalt, daß mindestens 50% aller Gruppen R′ Methylgruppen sind; Q einen aminfunktionellen Substituenten der Formel $-R''Z$ bedeutet, worin R″ ein divalenter Alkylenrest mit 3 bis 6 Kohlenstoffatomen oder ein Rest der Formel $-CH_2CH_2CH_2OCH_2-CHOHCH_2-$ ist und Z ein monovalenter Rest ausgewählt aus der Gruppe bestehend aus $-NR_2'''$, $-NR'''(CH_2)_nNR_2'''$ und

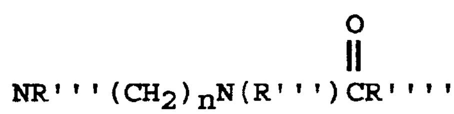

   worin R‴ Wasserstoff oder eine Alkylgruppe mit 1 bis 4 Kohlenstoffatomen bedeutet, R⁗ eine Alkylgruppe mit 1 bis 4 Kohlenstoffatomen bedeutet und n eine positive ganze Zahl von 2 bis 6 ist; z einen Wert von 0 oder 1 hat; x einen Durchschnittswert von 25 bis 3000 hat; y einen Durchschnittswert von 0 bis 100 hat, wenn z 1 ist und y einen Durchschnittswert von 1 bis 100 hat, wenn z 0 ist,

   (b) amidofunktionellen Polysiloxanen mit durchschnittlich 50 bis 1000 Siloxaneinheiten pro Molekül, wobei durchschnittlich 1 bis 50 Siloxaneinheiten pro Molekül amidgruppenhaltige Siloxaneinheiten sind, die einen Substituenten der Formel

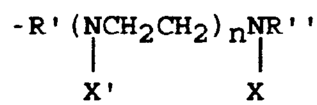

   tragen, worin n 0 oder 1 ist, R′ einen Alkylenrest mit 3 bis 6 Kohlenstoffatomen bedeutet und R″ einen Wasserstoffrest oder einen Alkylrest mit 1 bis 6 Kohlenstoffatomen bedeutet, X einen Acylrest der Formel

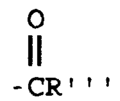

bedeutet, X′ Wasserstoff oder X bedeutet und R‴ einen Alkylrest mit 1 bis 4 Kohlenstoffatomen bedeutet und wobei im wesentlichen alle anderen organischen Substituenten in dem Polysiloxan Methylgruppen sind, und
(c) carboxyfunktionellen Polysiloxanen der Formel

$$QMe_2SiO(Me_2SiO)_x(MeRSiO)_ySiMe_2Q$$

worin Me ein Methylrest ist, R ein carboxyfunktioneller Rest ist, wobei der carboxyfunktionelle Rest ausgewählt ist aus der Gruppe bestehend aus Carboxyalkylresten und Carboxythioalkylresten, Q ausgewählt ist aus der Gruppe bestehend aus R, Me und OH-Gruppen, x einen Wert von 1 bis 1000 hat und y einen Wert von 1 bis 100 hat, zur Verbesserung der Beständigkeit eines Weichmachers auf der menschlichen Haut.

2. Verwendung nach Anspruch 1, worin die Mischung des Weichmachers und des Polysiloxans ein flüchtiges cyclisches Siloxanlösungsmittel einschließt.

**Revendications**

1. Utilisation d'un mélange d'une matière émolliente choisie dans le groupe consistant en huile minérale, huile de vison, huile de lanoline et pétrolatum, et d'une quantité efficace d'un composé organosilicié, et application du mélange au substrat à traiter, le composé organosilicié étant choisi parmi les groupes consistant en (a) les polysiloxanes comportant des fonctions amines de formule

$$R_{3-z}'Q_zSiO[R_2'SiO]_x[R'QSiO]_ySiQ_zR_{3-z}'$$

dans laquelle R′ désigne un groupe alcoyle de 1 à 4 atomes de carbone ou un groupe phényle, à la condition qu'au moins 50 pour cent de tous les groupes R′ soient des groupes méthyles; Q désigne un substituant aminé de formule -R″Z, dans laquelle R″ est un radical alkylène divalent de 3 à 6 atomes de carbone ou un radical de formule $-CH_2CH_2CH_2OCH_2-CHOHCH_2-$ et Z est un radical monovalent choisi dans le groupe consistant en -NR,‴, $-NR'''(CH_2)_nR_2'''$; et

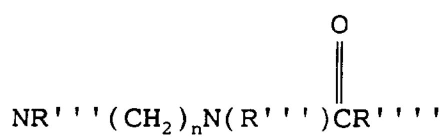

dans laquelle R‴ désigne l'atome d'hydrogène ou un groupe alcoyle de 1 à 4 atomes de carbone, R⁗ désigne un groupe alcoyle de 1 à 4 atomes de carbone et n est un nombre entier positif de 2 à 6; z est égal à 0 ou 1; x a une valeur moyenne de 25 à 3000; y a une valeur moyenne de 0 à 100 lorsque z est égal à 1, et y a une valeur moyenne de 1 à 100 lorsque z est égal à 0, (b) les polysiloxanes comportant des fonctions amides ayant en moyenne de 50 à 1000 unités siloxanes par molécule, 1 à 50 unités siloxanes par molécule en moyenne étant des unités siloxanes amides portant un substituant de formule

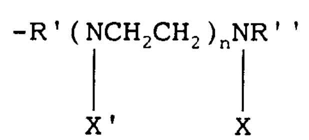

dans laquelle n est 0 ou 1, R′ désigne un radical alkylène de 3 à 6 atomes de carbone, et R″ désigne l'atome d'hydrogène ou un radical alcoyle de 1 à 6 atomes de carbone, X désigne un radical acyle de formule

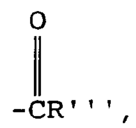

X' désigne l'atome d'hydrogène ou X, et R''' désigne un groupe alcoyle de 1 à 4 atomes de carbone, et pratiquement tous les autres substituants organiques dans le polysiloxane étant des groupes méthyle, et (f) les polysiloxanes comportant des fonctions carboxyles de formule

$$QMe_2SiO(Me_2SiO)_x(MeRSiO)_ySiMe_2Q$$

dans laquelle Me est un radical méthyle, R est un groupe à fonction carboxyle, ledit groupe à fonction carboxyle étant choisi parmi le groupe constitutif des radicaux carboxyalcoyles et carboxythioalcoyles, Q étant choisi parmi le groupe constitué des groupes R, Me et OH, x a une valeur de 1 à 1000, et y a une valeur de 1 à 100, pour améliorer la durabilité de l'émollient sur la peau humaine.

2. Utilisation selon la Revendication 1 dans laquelle le mélange de l'émollient et du polysiloxane comprend un siloxane cyclique volatil en tant que solvant.